# EUROPÄISCHE PATENTSCHRIFT

(11) **EP 0 761 681 B1**
(45) Veröffentlichungstag und Bekanntmachung des Hinweises auf die Patenterteilung: **13.03.2002**
(21) Anmeldenummer: 96113530.8
(22) Anmeldetag: 22.08.1996
(51) Int. Cl.: C07K 5/06, C07K 14/00

(54) **Verfahren zur Herstellung substituierter N-Ethyl-Glycinderivate**
Process for the preparation of substituted derivatives of N-ethylglycine
Procédé de préparation de dérivés de substitués de N-éthylglycine

(30) Priorität: 04.09.1995 DE 19532553
(43) Veröffentlichungstag der Anmeldung: 12.03.1997
(73) Patentinhaber: HOECHST AKTIENGESELLSCHAFT, 65929 Frankfurt am Main (DE)
(72) Erfinder: Breipohl, Gerhard, Dr., 60529 Frankfurt (DE); Uhlmann, Eugen, Dr., 61479 Glashütten (DE); Will, David William, Dr., 65760 Eschborn (DE)

(56) Entgegenhaltungen:
- EP-A- 0 672 661
- EP-A- 0 672 677
- WO-A-96/40709
- TETRAHEDRON, Bd. 51, Nr. 22, 29.Mai 1995, OXFORD GB, Seiten 6179-6194, XP002030024 S A THOMSON ET AL. : "FMOC mediated synthesis of peptide nucleic acids"
- JOHN JONES: "The chemical synthesis of peptides" 1991 , CLARENDON PRESS , OXFORD, ENGLAND XP002030025 639191 *Seiten 22-23 und 26-27 in Zusammenhang mit Seiten 34-35*

## Beschreibung

Die vorliegende Erfindung betrifft ein neues, verbessertes Verfahren zur Herstellung von substituierten N-Ethyl-Glycinderivaten, die in EP-A 672 661 beschrieben sind. Diese substituierten N-Ethyl-Glycinderivate dienen zur Herstellung von PNA und PNA-/DNA-Hybriden wie sie in EP-A 672 677 beschrieben sind. Deren Anwendung bezieht sich auf die Verwendung als Inhibitoren der Genexpression (Antisense Oligonucleotide, Ribozyme, Sense Oligonucleotide und Triplex Forming Oligonucleotide), als Sonden zum Nachweis von Nucleinsäuren und als Hilfsmittel in der Molekularbiologie. Von Thomson et al. (Tetrahedron 51 : 6179-6194, 1995) wurde bereits die Synthese von F-moc-geschützten PNA-Monomeren beschrieben.

Ziel der Erfindung ist es, ein einfaches, preiswertes Verfahren zur Herstellung dieser substituierten N-Ethyl-Glycinderivate zu finden.

Das neuartige Verfahren zur Herstellung der substituierten N-Ethyl-Glycinderivate der Formel I 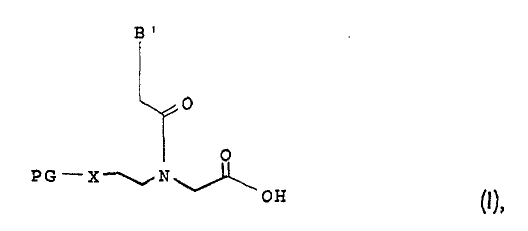 worin
- PG: für eine gegen schwache Säuren labile Aminoschutzgruppe vom Urethantyp, wie z.B. 1-(1-Adamantyl)1-methylethoxycarbonyl (Adpoc), 1-(3,5-di-tert.-butylphenyl)1-methylethoxycarbonyl (t-Bumeoc) und 1-Methyl-1-(4-biphenyl)ethyloxycarbonyl(Bpoc), 3,5-Dimethoxyphenyl-2-propyl-2-oxycarbonyl (Ddz) oder vom Trityl-Typ, wie Triphenylmethyl (Trt), (4-Methoxyphenyl)diphenylmethyl (Mmt), (4-Methylphenyl)diphenylmethyl (Mtt), Di-(4-methoxyphenyl)phenylmethyl (Dmt) und 9-(9-Phenyl)xanthenyl (Pixyl),
- X: für NH oder O, und
- B': für in der Nucleotidchemie übliche Basen, beispielsweise für natürliche Basen wie Adenin, Cytosin, Guanin, Thymin und Uracil oder unnatürliche Basen, wie Purin, 2.6-Diaminopurin, 7-Deazaadenin oder 7-Deazaguanin, die beide gegebenenfalls in der 7-Position durch einen Substituenten aus der Reihe Halogen, (C₁-C₁₀)-Alkyl, (C₂-C₁₀)-Alkenyl oder (C₃-C₁₀)-Alkinyl, bevorzugt Chlor, Brom oder Jod, (C₃-C₆)-Alkyl, (C₃-C₆)-Alkenyl oder (C₃-C₆)-Alkinyl, substituiert sind, N⁴N⁴-Ethanocytosin, N6N6-Ethano-2.6-diaminopurin, 5-Methylcytosin,5-(C₃-C₆)-Alkinyl-uracil, 5-(C₃-C₆)-Alkinyl-cytosin, 5-Fluor-uracil oder Pseudoisocytosin, deren exocyclische Amino bzw. Hydroxygruppen durch geeignete bekannte Schutzgruppen, wie die Benzoyl-, Isobutanoyl-, Acetyl-, Phenoxyacetyl-, 4-(t-Butyl)-benzoyl-, 4-(t-Butyl)-Phenoxyacetyl-, 4-(Methoxy)-benzoyl-, 2-(4-Nitrophenyl)ethyl-oxycarbonyl-, 2-(2,4-Dinitrophenyl)ethyl-oxycarbonyl-, 9-Fluorenylmethoxycarbonyl- Diphenylcarbamoyl oder Formamidin-Gruppe, bevorzugt die Benzoyl-, Isobutanoyl-, Acetyl-, Phenoxyacetyl-, 4-(t-Butyl)-benzoyl-, 4-(Methoxy)-benzoyl-Gruppe, und für Guanin auch durch eine Kombination von 2-N-Acetyl mit 6-0-Diphenylcarbamoyl geschützt sind, stehen
sowie deren Salze, bevorzugt deren Salze mit tert. organischen Basen, wie z. B. Triethylamin oder Pyridin,
ist dadurch gekennzeichnet, daß man
a) eine Verbindung der Formel II 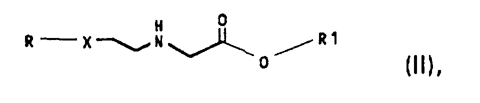 worin
   - R: Wasserstoff, oder wenn X = NH ist, eine säurelabile Schutzgruppe, wie z. B. Boc, Ddz, Trt, bevorzugt Boc, und
   - R¹: eine gegen Säuren labile und gegen Amine stabile Schutzgruppe, wie z. B. tert. Butyl und (2-Chlorphenyl)diphenylmethyl, bevorzugt tert.-Butyl, bedeuten, und
   - x: wie oben definiert ist,
   mit einer Verbindung der Formel III 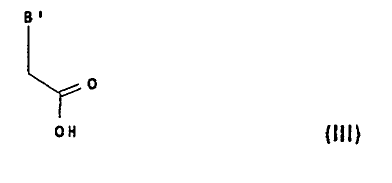 worin
   - B': wie oben definiert ist,
   bei 0 - 45°C, bevorzugt bei Raumtemperatur, in einem geeigneten Lösungsmittel, wie z. B. DMF, Acetonitril, Dichlormethan oder Mischungen dieser Lösungsmittel unter Verwendung eines in der Peptidchemie üblichen Kupplungsreagenzes, wie z. B. Carbodiimiden, Phosphonium-Reagenzien, Uronium-Reagenzien, Säurehalogeniden oder aktivierten Estern,
   zu einer Verbindung der Formel IV 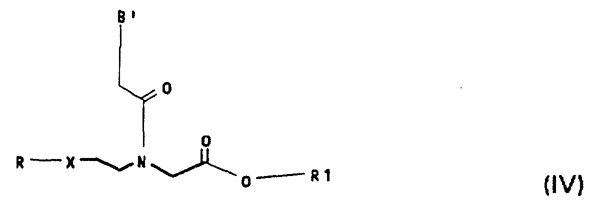 worin
   - R, X, B' und R¹: wie oben definiert sind,
   umsetzt,
b) anschließend die Verbindung der Formel IV durch Abspaltung der säurelabilen Esterschutzgruppe R¹ bzw. für X = NH durch gleichzeitige Abspaltung der säurelabilen Schutzgruppe R unter geeigneten sauren Bedingungen mit z. B. Trifluoressigsäure in einem geeigneten Lösungsmittel wie z. B. Dichlormethan, Ethylacetat, Dioxan etc. gegebenenfalls unter Zusatz von Kationenfängern wie z. B. Anisol, Thiophenol, Triethylsilan etc.,
   in eine Verbindung der Formel V überführt, wobei bei Verbindungen der Formel IV mit X = O im Laufe dieser Abspaltung in gewissem Maße auch das Lacton der Formel Va entstehen kann, das aber durch Behandlung mit Basen, wie z. B. NaOH oder Triethylamin, in wäßrigem Medium einfach in das offenkettige Derivat der Formel V überführt werden kann, 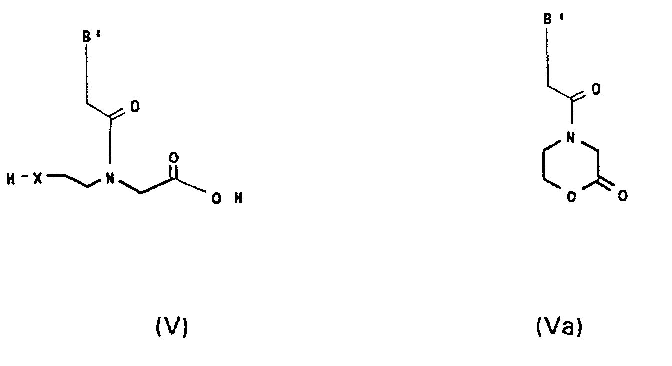 worin
   - X und B': wie oben definiert sind,
c) die Verbindung der Formel V mit einem geeigneten Reagenz, wie z. B. t-Bumeoc-fluorid, Adpoc-azid, Bpoc-azid, Ddz-(phenyl)carbonat, Trt-Cl, Mtt-Cl, Mmt-CI, Dmt-CI oder Pixyl-Cl, in einem geeigneten Lösungsmittel, wie z. B. DMF, NMP, Acetonitril, Dichlormethan oder Mischungen dieser Lösungsmittel unter Verwendung einer Hilfsbase z. B. NEM, DIPEA, Pyridin, Triethylamin in die Verbindung der Formel I und anschließend gegebenenfalls in deren Salze überführt.

In der Peptidsynthese übliche Aktivierungsmethoden sind z. B. in Houben-Weyl, Methoden der organischen Chemie, Band 15/2 oder weitere Reagenzien, wie z. B. BOP (B. Castro, J. R. Dormoy, G. Evin and C. Selve, Tetrahedron Lett. 1975, 1219-1222), PyBOP (J. Coste, D. Le-Nguyen und B. Castro, Tetrahedron Lett. 1990, 205-208), BroP (J. Coste, M.-N. Dufour, A. Pantaloni und B. Castro, Tetrahedron Lett. 1990, 669-672), PyBroP (J. Coste, E. Frerot, P. Jouin und B. Castro, Tetrahedron Lett. 1991, 1967-1970) und Uronium-Reagenzien, wie z. B. HBTU (V. Dourtoglou, B. Gross, V. Lambropoulou, C. Zioudrou, Synthesis 1984, 572-574), TBTU, TPTU, TSTU, TNTU, (R. Knorr, A. Trzeciak, W. Bannwarth and D. Gillessen, Tetrahedron Letters 1989, 1927-1930), TOTU (EP-A-0 460 446), HATU (L. A. Carpino, J. Am. Chem. Soc. 1993, 115, 4397-4398), HAPyU, TAPipU (A. Ehrlich, S. Rothemund, M. Brudel, M. Beyermann, L. A. Carpino und M. Bienert, Tetrahedron Lett. 1993, 4781-4784), BOI (K. Akaji, N. Kuriyama, T. Kimura, Y. Fujiwara und Y. Kiso, Tetrahedron Lett. 1992, 3177-3180), Propanphosphonsäureanhydrid (PPA) (H. Wissmann, H. J. Kleiner, Angew. Chem. 92, 129-130, 1990) oder Säurechloride bzw. Säurefluoride (L. A. Carpino, H. G. Chao, M. Beyermann and M. Bienert, J. Org. Chem., 56(1991), 2635; J.-N. Bertho, A. Loffet, C. Pinel, F. Reuther and G. Sennyey in E. Giralt and D. Andreu (Eds.) Peptides 1990, Escom Science Publishers B. V.1991, pp. 53-54; J. Green und K. Bradley, Tetrahedron 1993, 4141-4146), 2,4,6-Mesitylensulfonyl-3-nitro-1,2,4-triazolid (MSNT) (B. Blankemeyer-Menge, M. Nimitz und R. Frank, Tetrahedron Lett. 1990, 1701-1704), 2,5-Diphenyl-2,3-dihydro-3-oxo-4-hydroxythiophendioxid (TDO) (R. Kirstgen, R. C. Sheppard, W. Steglich, J. Chem. Soc. Chem. Commun. 1987, 1870-1871) oder aktivierte Ester (D. Hudson, Peptide Res. 1990, 51 - 55) in den jeweiligen Literaturstellen beschrieben.

Bevorzugt ist die Verwendung von Carbodiimiden, z. B. Dicyclohexylcarbodiimid oder Diisopropylcarbodiimid. Bevorzugt verwendet werden ebenfalls Phosphonium-Reagenzien, wie z. B. PyBOP oder PyBroP, Uronium-Reagenzien, wie z. B. HBTU, TBTU, TPTU, TSTU, TNTU, TOTU oder HATU, BOI oder Säurechloride bzw. Säurefluoride sowie Propanphosphonsäureanhydrid (PPA).

Der Vorteil des neuen Verfahrens für die Herstellung der Verbindungen der Formel I mit X = NH liegt darin, daß aus der Zwischenstufe der Formel IV durch Einwirkung eines einzigen Reagenzes z. B. Trifluoressigsäure gleichzeitig die Schutzgruppen für die primäre Aminofunktion und die Carboxylgruppe des Aminoethylglycins abgespalten werden. Durch Einführung der Schutzgruppe PG entsteht dann direkt die Verbindung der Formel I. Für die Herstellung der Titelverbindungen der Formel I mit X = O liegt der Vorteil darin, daß durch den Schutz der Carboxylgruppe sich zum einen die Löslichkeit des Hydroxyethylglycinderivates in organischen Lösungsmittels verbessert und zum anderen die Verknüpfung mit Verbindungen der Formel III erleichtert.

Die Synthese der Verbindungen der Formel II erfolgt z. B. durch die Umsetzung von kommerziell erhältlichem 1,2-Diaminoethan oder Aminoethanol mit den entsprechenden Halogenessigsäureester wie z. B. kommerziell verfügbarem Chloressigsäure tert.-butylester, Bromessigsäure tert.-butylester. Für X = NH und R = säurelabile Schutzgruppe folgt eine Umsetzung des Aminoethylglycinesters mit einem geeigneten Schutzgruppenreagenz wie z. B. Ddz-(phenyl)carbonat, Trt-Cl, Di-tert.butyldicarbonat, Boc-ONSu, tert.-Butylphenylcarbonat. Ein weiteres Verfahren besteht in der Umsetzung von Mono-geschütztem 1,2-Diaminoethan, wie z. B. Mono-Boc-diaminoethan mit Halogenessigsäureestern, wie z. B. Chloressigsäure tert.-butylester oder Bromessigsäure tert.-butylester.

Ein weiteres Verfahren zur Herstellung der Verbindungen der Formel II mit R = Boc und R¹ = tert.-Butyl besteht in der reduktiven Aminierung von Glyoxylsäure-tert.-butylester mit Ethylendiamin, Mono-Boc-Ethylendiamin (für X = NH) bzw. Aminoethanol (für X = O) mit Wasserstoff an Palladium auf Kohle oder mit Natriumcyanoborhydrid oder Natrium-triacetoxy-borhydrid als Reduktionsmittel. Der für diese Reaktion notwendige Glyoxylsäure-tert.-butylester kann wie z. B. in J. E. Bishop, J. F. O'Connell, H. Rapoport, J. Org. Chem. 1991, 5079-5091 beschrieben erhalten werden. Die Synthese von Mono-Boc-Ethylendiamin ist z. B. in Kapcho, A. P.; Kuell, C. S.; Synth Commun. (1990), 2559-2564 beschrieben.

Ebenfalls geeignet für die Herstellung von Verbindungen der Formel II mit X = NH ist die reduktive Aminierung von Boc-Glycinal mit Glycin-tert.-butylester mit Wasserstoff an Palladium auf Kohle oder mit Natriumcyanoborhydrid oder Natrium-triacetoxy-borhydrid als Reduktionsmittel. Das dazu notwendige Boc-Glycinal wird z. B. wie bei A. Evidente, G. Picalli, A. Sisto, M. Ohba, K. Honda, T. Fujii, Chem. Pharm. Bull. 1992, 1937-1939 beschrieben hergestellt, Glycin-tert.-butylester ist kommerziell erhältlich.

Die Nucleobasen-Essigsäurederivate der Formel III können durch Alkylierung der ensprechenden Nucleobasen oder der in der exocyclischen Hydroxy- oder Aminofunktion geschützten Nucleobasen mit Chloressigsäure, Bromessigsäure, Jodessigsäure oder deren Estern erhalten werden. Gegebenenfalls werden dabei zur selektiven Alkylierung zusätzlich temporäre Schutzgruppen an der Nucleobase eingeführt. Als Schutzgruppe für die Nucleobasen können alle mit der gegen schwache Säuren labilen Schutzgruppe PG kompatible Schutzgruppen verwendet werden. Für die exocyclische Aminofunktion werden bevorzugt Schutzgruppen, wie die Benzoyl-, Isobutanoyl-, Acetyl-, Phenoxyacetyl-, 4-(t-Butyl)-benzoyl-, 4-(t-Butyl)-Phenoxyacetyl-, 4-(Methoxy)-benzoyl-, 2-(4-Nitrophenyl)ethyl-oxycarbonyl-, 2-(2,4-Dinitrophenyl)ethyl-oxycarbonyl-, 9-Fluorenylmethoxycarbonyl- oder Formamidin-Gruppe verwendet. Insbesondere bevorzugt sind die Benzoyl-, Isobutanoyl-, 4-(t-Butyl)-benzoyl-, 2-(4-Nitrophenyl)ethyl-oxycarbonyl-, 2-(2,4-Dinitrophenyl)ethyl-oxycarbonyl-, 9-Fluorenylmethoxycarbonyl-, 4-(Methoxy)-benzoyl- oder para-(t-Butyl)-Phenoxyacetyl-, para-Nitrophenyl-2-ethyl-oxycarbonyl-Gruppe.

Gegenstand der Erfindung sind ferner die wertvollen Zwischenprodukte der Formel IV, worin R, X, B' und R¹ wie oben definiert sind, worin bevorzugt jedoch R Wasserstoff oder wenn X = NH ist, Boc und R¹ tert. Butyl bedeutet.

### Beispiele:

### Beispiel 1:

### Hydroxyethylglycin-tert.-butylester

### (H-Oeg-OtBu)

30.2 ml (0.5 Mol) Aminoethanol werden in 200 ml DMF gelöst, 17 ml (0.1 Mol) Diisopropylethylamin zugegeben und 14.8 ml (0.1 Mol) Bromessigsäure-tert.-butylester zugetropft. Die Mischung wird 24 h bei Raumtemperatur gerührt und dann das Lösungsmittel am Rotationsverdampfer im Vakuum abgedampft. Der Rückstand wird in 100 ml Wasser aufgenommen, mit Natriumchlorid gesättigt und mit dreimal 100 ml Ethylacetat extrahiert. Die organische Phase wird mit wenig gesättigter NaCl-Lösung gewaschen, über Natriumsulfat getrocknet und dann zur Trocknene eingeengt. Man erhält 11.96 g des Produkts als farbloses Öl.
MS(ES⁺): 176.2 (M+H)⁺
R_{f} =0.51 (2-Butanon:Wasser:Pyridin:Essigsäure/70:15:15:2)

### Beispiel 1a:

### Hydroxyethylglycin-tert.-butylester

### (H-Oeg-OtBu)

3 ml (0.05 Mol) Aminoethanol werden in 20 ml DMF gelöst 1.7 ml (0.01 Mol) Diisopropylethylamin zugegeben und 1.4 ml (0.01 Mol) Chloressigsäure-tert.-butylester zugetropft. Die Mischung wird 24 h bei Raumtemperatur gerührt und dann das Lösungsmittel am Rotationsverdampfer im Vakuum abgedampft. Der Rückstand wird in 20 ml Wasser aufgenommen, mit Natriumchlorid gesättigt und mit dreimal 20 ml Ethylacetat extrahiert. Die organische Phase wird mit wenig gesättigter NaCl-Lösung gewaschen, über Natriumsulfat getrocknet und dann zur Trocknene eingeengt. Man erhält 1.9 g des Produkts als farbloses Öl.
MS(ES⁺): 176.2 (M+H)⁺
R_{f} = 0.51 (2-Butanon:Wasser:Pyridin:Essigsäure/70:15:15:2)

### Beispiel 2:

### N-(Hydroxy)ethyl-N-((1-thyminyl)acetyl)glycin-tert.-butylester

### (H-Oeg(T)-OtBu)

8.9 g (53 mMol) H-Oeg-OtBu werden in 100 ml DMF gelöst, dann nacheinander 8.8 g (48 mMol) Thyminylessigsäure, 19.4 ml (106 mMol) Triethylamin und 17.4 g (53 mMol) TOTU hinzugegeben. Es wird noch 3h bei Raumtemperatur gerührt, dann das Lösungsmittel am Rotationsverdampfer im Vakuum abgedampft. Der Rückstand wird mit wenig Ethylacetat verrührt, wobei das Produkt auszufallen beginnt. Man läßt noch über Nacht bei 4°C stehen, saugt das ausgefallene Produkt ab, wäscht mit wenig Ethylacetat nach und trocknet im Vakuum. Ausbeute: 11.8 g farblose Substanz.
MS(ES⁺): 342.2 (M+H)⁺
R_{f} = 0.75 (2-Butanon:Wasser:Pyridin:Essigsäure/70:15:15:2)

### Beispiel 3:

### N-(Hydroxy)ethyl-N-((1-thyminyl)acetyl)glycin

### (H-Oeg(T)-OH)

9.47 g (28 mMol) H-Oeg(T)-OtBu werden in 150 ml Dichlormethan suspendiert und mit 100 ml 95% Trifluoressigsäure (5% Wasser) versetzt. Es ensteht ein klare Lösung, die 3h bei Raumtemperatur gerührt wird. Das Reaktinsgemisch wird dann in 1l kalten (0°C) gut gerührten Methyl-tert.butylether eingetropft, wobei das Produkt ausfällt. Das ausgefallene Rohprodukt (enthält auch etwas entstandenes Lacton) wird in einer Mischung aus 170 ml Dioxan, 170 ml Wasser und 6.4 ml Triethylamin gelöst und 2h bei Raumtemperatur gerührt, wobei das Lacton geöffnet wird und das Triethylammonium-Salz entsteht. Dann wird das Gemisch zur Trockene eingedampft und der Rückstand im Vakuum getrocknet.
Ausbeute: 10.51 g amorpher Feststoff
MS(ES⁺): 286.2 (M+H)⁺
R_{f} =0.12 (Dichlormethan:Methanol:Ethylacetat/10:2:1 und 1% Triethylamin)

### Beispiel 4:

### N-(Di-(4-Methoxyphenyl)-phenylmethyloxy)ethyl-N-((1-thyminyl)acetyl)glycin

### (Dmt-Oeg(T)-OH)

1.0 g (2.6 mMol) H-Oeg(T)-OH NEt₃ werden in 10 ml DMF gelöst und 1.4 ml (10 mMol) Triethylamin zugegeben. Dann werden 1.8 g (5.2 mMol) Dmt-CI in 10 ml Dichlormethan zugetropft und die Mischung 16 h bei Raumtemperatur gerührt. Dann wird das Lösungsmittel am Rotationsverdampfer im Vakuum abgedampft, der Rückstand in Dichlormethan aufgenommen, mit Wasser gewaschen, die organische Phase über Natriumsulfat getrocknet und dann eingedampft. Das erhalten Rohprodukt wird mittels Säulenchromatographie an Kieselgel mit Dichlormethan/Methanol/Ethylacetat 15:1:1 mit 1% Triethylamin als Elutionsmittel gereinigt. Die das Produkt enthaltenden Fraktionen werden vereinigt und eingeengt. Man erhält 96C mg des Produktes als Schaum.
MS(FAB, MeOH/NBA): 587.3 (M)⁺
R_{f} = 0.29 (Dichlormethan:Methanol:Ethylacetat/10:2:1 und 1% Triethylamin)

### Beispiel 5:

### N-(Hydroxy)ethyl-N-((1-(N⁴-(4-tert-butylbenzoyl)-cytosyl)acetyl)glycin-tert.-butylester

### (H-Oeg(C^{MeOBz})-OtBu)

1.8 g (11 mMol) H-Oeg-OtBu werden in 100 ml DMF gelöst, dann nacheinander 3.0 g (10 mMol) N⁴-(4-Methoxybenzoyl)-N¹-carboxymethyl-cytosin, 3 ml Triethylamin und 3.6 g (11 mMol) TOTU hinzugegeben. Es wird noch 4h bei Raumtemperatur gerührt, dann das Lösungsmittel am Rotationsverdampfer im Vakuum abgedampft. Der Rückstand wird in Ethylacetat aufgenommen und zweimal mit Natriumhydrogencarbonat-Lösung gewaschen. Dabei fällt das Produkt in der Ethylacetatphase aus. Man saugt das ausgefallene Produkt ab, wäscht mit wenig Ethylacetat nach und trocknet im Vakuum. Ausbeute: 3.06 g farblose Substanz.
MS(FAB, MeOH/NBA): 461.3 (M+H)⁺
R_{f} =0.81 (2-Butanon:Wasser:Pyridin:Essigsäure/70:15:15:2)

### Beispiel 6:

### N-(Hydroxy)ethyl-N-((1-(N⁴-(4-tert-butylbenzoyl)-cytosyl)acetyl)glycin

### (H-Oeg(C^{MeOBz})-OH)

1.5 g (3.4 mMol) H-Oeg(C^{MeOBz})-OtBu werden in einer Mischung aus 30 ml Dichlormethan und 20 ml 95% Trifluoressigsäure (5% Wasser) die 2.5 ml Anisol enthält, gelöst. Die klare Lösung wird 5h bei Raumtemperatur gerührt und dann in 500 ml kalten (0°C) gut gerührten Methyl-tert.butylether eingetropft, wobei das Produkt ausfällt. Das ausgefallene Rohprodukt (enthält auch etwas entstandenes Lacton) wird in einer Mischung aus 25 ml Dioxan, 25 ml Wasser und 0.44 ml Triethylamin gelöst und 3h bei Raumtemperatur gerührt, wobei das Lacton geöffnet wird und das Triethylammonium-Salz entsteht. Dann wird das Gemisch zur Trockene eingedampft und der Rückstand mittels Säulenchromatographie an Kieselgel mit Dichlormethan/Methanol/Ethylacetat 10:3:2 mit 1% Triethylamin als Elutionsmittel gereinigt.
Ausbeute: 920 mg amorpher Feststoff
MS(ES⁺): 405.2 (M+H)⁺
R_{f} =0.16 (Dichlormethan:Methanol:Ethylacetat/10:3:2 und 1% Triethylamin)

### Beispiel 7:

### N-(Di-(4-Methoxyphenyl)-phenylmethyloxy)ethyl-N-((1-(N⁴-(4-Methoxylbenzoyl)-cytosyl)acetyl)glycin

### (Dmt-Oeg(C^{MeBz})-OH)

920 mg (1.8 mMol) H-Oeg(C^{MeBz})-OH · NEt₃ werden in 10 ml DMF gelöst und 1ml (7.2 mMol) Triethylamin zugegeben. Dann werden 1.2 g (3.6 mMol) Dmt-CI in 10 ml Dichlormethan zugetropft und die Mischung 16 h bei Raumtemperatur gerührt. Dann wird das Lösungsmittel am Rotationsverdampfer im Vakuum abgedampft, der Rückstand in Dichlormethan aufgenommen, mit Wasser gewaschen, die organische Phase über Natriumsulfat getrocknet und dann eingedampft. Das erhaltene Rohprodukt wird mittels Säulenchromatographie an Kieselgel mit Dichlormethan/Methanol/Ethylacetat 15:1:1 mit 1% Triethylamin als Elutionsmittel gereinigt. Die das Produkt enthaltenden Fraktionen werden vereinigt und eingeengt. Man erhält 900 mg des Produktes als Schaum.
MS(FAB, MeOH/NBA): 707.3 (M+H)⁺
R_{f} = 0.24 (Dichlormethan:Methanol:Ethylacetat/10:3:2 und 1 % Triethylamin)

### Beispiel 8:

### N-(hydroxy)ethyl-N-(9-(N⁶-(4-methoxybenzoyl)-adenosyl)acetyl)glycin-tert.-butylester

### (H-Oeg(A^{MeOBz})-OtBu)

534 mg (3.3 mMol) H-Oeg-OtBu worden in 10 ml DMF gelöst, dann nacheinander, 1.0g (3.1 mMol) N⁶-(4-Methoxybenzoyl)-N⁹-carboxymethyladenin, 0.77 ml (4.5 mMol) N-Ethylmorpholin und 1.0 g (3.1 mMol) TOTU hinzugegeben. Es wird über Nacht bei Raumtemperatur gerührt, dann das Lösungsmittel im Vakuum am Rotationsverdampfer abgedampft. Der Rückstand wird in Ethylacetat aufgenommen und zweimal mit Natriumhydrogencarbonat-Lösung und Wasser gewaschen. Die organische Phase wird über Natriumsulfat getrocknet und dann eingedampft. Man erhält 1.33 g des Produktes.
MS(FAB, MeOH/NBA): 485.3 (M+H)⁺
Rf = 0.63 (n-Butanol:Wasser:Essigsäure/3:1:1)

### Beispiel 9:

### N-(Hydroxy)ethyl-N-(9-(N⁶-(4-methoxybenzoyl)-adenosyl)acetyl)glycin

### (H-Oeg(A^{MeOBz})-OH)

1.3 g (2.7 mMol) H-Oeg(A^{MeOBz})-OtBu werden in 15 ml 95% Trifluoressigsäure (5% Wasser) gelöst. Die klare Lösung wird 1h bei Raumtemperatur gerührt und dann in 250 ml kalten (0°C) gut gerührten Diethylether eingetropft, wobei das Produkt ausfällt. Das ausgefallene Rohprodukt (enthält auch etwas entstandenes Lacton) wird in einer Mischung aus 15 ml Dioxan, 15 ml Wasser und 0.4 ml Triethylamin gelöst und 2 h bei Raumtemperatur gerührt, wobei das Lacton geöffnet wird und das Triethylammonium-Salz entsteht. Dann wird das Gemisch zur Trockene eingedampft und 3mal mit Pyridin abge aucht. Der Rückstand wird direkt in die nächste Reaktion eingesezt.
MS(FAB, NBA/MeOH/LiCl): 435.2 (M+Li)⁺
Rf = 0.37 (n-Butanol:Wasser:Essigsäure/3:1:1)

### Beispiel 10:

### N-(Di-(4-Methoxyphenyl)-phenylmethyloxy)ethyl-N-(9-(N⁶-(4-methoxybenzoyl)-adenosyl)acetyl)glycin

### (Dmt-Oeg(A^{MeOBz})-OH)

Das in der vorherigen Reaktion erhaltene H-Oeg(A^{MeOBz})-OH · NEt₃ wird in 10ml Pyridin gelöst. Dann werden 1.7 g ( 5 mMol) Dmt-CI zugegeben und die Mischung 16 h bei Raumtemperatur gerührt. Dann wird das Lösungsmittel am Rotationsverdampfer im Vakuum abgedampft, der Rückstand in Dichlormethan aufgenommen. Man wäscht mit 5%ige wässriger Zitronensäure und ges. wässriger NaCI Lösung, trocknet die organische Phase über Natriumsulfat und engt dann zur Trockene ein. Das erhaltene Rohprodukt wird mittels Säulenchromatographie an Kieselgel mit Dichlormethan mit einem Gradienten von 1-10% Methanol 1% Triethylamin als Elutionsmittel gereinigt. Die das Produkt enthaltenden Fraktionen werden vereinigt und eingeengt. Der Rückstand wird in 5ml Dichlormethan gelöst und dann in 100 ml gut gerührten Diethylether eingetropft, wobei das Produkt ausfällt. Man erhält 580 mg des Produktes als Pulver.
MS(FAB, MeOH/NBA): 731.3 (M+H)⁺
Rf = 0.70 (n-Butanol:Wasser:Essigsäure/3:1:1)

### Beispiel 11:

### N-(tert.Butyloxycarbonylaminoethyl)glycin-tert.butylester

### Boc-Aeg-OtBu

### Stufe 1:

In einen Dreihalskolben mit Rührer, Rückflußkühler und Tropftrichter werden 120 ml (1.8 Mol) 1,2-Diaminoethan in 300 ml Dichlormethan gegeben. Zur gut gerührten Lösung werden innerhalb von ca. 2.5h 42.7 ml (0.3 Mol) Chloressigsäure-tert.-butylester in 100 ml Dichlormethan zugetropft. Die Reaktion ist leicht exotherm. Nach ca. 15 min tritt eine Trübung ein und das gebildete Diaminhydrochlorid setzt sich als ölige Phase ab. Zur Vervollständigung der Reaktion wird noch ca. 2h bei Raumtemperatur nachgerührt (DC Kontrolle in Ethylmethylketon/Pyridin/Wasser/Eissessig 70:15:15:2) und dann das Reaktionsgemisch mit 200 ml Wasser versetzt. Die organische Phase wird abgetrennt und die Wasserphase noch zweimal mit je 100 ml Dichlormethan extrahiert. Die vereinigten organischen Phasen werden dann noch einmal mit 100 ml Wasser rückextrahiert und über Natriumsulfat getrocknet. Nach Abfiltrieren des Trockenmittels wird die organische Phase auf 800 ml aufgefüllt und zur Bestimmung der Rohausbeute 40 ml zur Trockene eingeengt. Rückstand 2.28 g, entspricht Gesamtmenge von 45.6 g (86%). Diese Dichlormethan-Lösung des Rohproduktes wird dann wie nachfolgend beschrieben direkt weiter umgesetzt zum Boc-Aeg-OtBu.
MS(ES⁺): 189.1 (M+H)⁺
R_{f} = 0.36 (2-Butanon:Wasser:Pyridin:Essigsäure/70:15:15:2)

### Stufe 2:

0.26 Mol H-Aeg-OtBu (Lösung in 800 ml Dichlormethan) werden mit 38 ml (0.27 Mol) Triethylamin versetzt und dann unter gutem Rühren langsam innerhalb von 45 min eine Lösung von 54.5 g (0.25 Mol) Di-tert.-butyldicarbonat in 100 ml Dichlormethan zugetropft. Dabei wird die Reaktionlösung durch entstehendes Carbamat (aus CO₂) dickflüssiger und daher mit 100 ml Dichlormethan verdünnt. Nach beendeter Reaktion ist die Lösung dünnflüssig und nur schwach getrübt. Es werden 100 ml Wasser zugegeben, die organische Phase wird abgetrennt und die wäßrige Phase nochmals mit 100 ml Dichlormethan extrahiert. Die organische Phase wird über Natriumsulfat getrocknet und dann im Vakuum eingeengt. Rohprodukt: 77.6 g eines farblosen Öls. Dieses Rohprodukt wird durch Säulenchromatographie an Kieselgel mit Ethylacetat als Elutionsmittel gereinigt. Ausbeute: 48.1 g
MS(ES⁺): 275.2 (M+H)⁺
R_{f} =0.46 (Ethylacetat)

### Beispiel 12:

### N-(tert.Butyloxycarbonylaminoethyl)-N-((1-thyminyl)acetyl)glycin-tert.butylester

### Boc-Aeg(T)-OtBu

1.375 g (5 mMol) Boc-Aeg-OtBu werden in 20 ml DMF gelöst und dann nacheinander 921 mg (5mMol) Thyminylessigsäure, 1.64 g(5mMol) TOTU und 1.7 ml (10mMol) Diisopropylethylamin zugegeben. Die Mischung wird über Nacht bei Raumtemperatur gerührt und dann im Vakuum eingedampft. Der ölige Rückstand wird in 70 ml Ethylacetat aufgenommen und jeweils 5mal mit je 5 ml Natriumhydrogencarbonat-Lösung bzw. Kaliumhydrogensulfat-Lösung extrahiert. Dann wird noch dreimal mit je 5 ml Wasser gewaschen. Die organische Phase wird über Natriumsulfat getrocknet und dann zur Trockene eingeengt. Der verbliebene feste Rückstand wird mit Diethylether verrieben, abgesaugt und getrocknet. Ausbeute: 1.82 g
MS(ES⁺): 441.3 (M+H)⁺
R_{f} = 0.55 (Dichlormethan:Methanol:Triethylamin/100:10:1)

### Beispiel 12a:

### N-(tert.Butyloxycarbonylaminoethyl)-N-((1-thyminyl)acetyl)glycin-tert.butylester

### Boc-Aeg(T)-OtBu

6.875 g (25 mMol) Boc-Aeg-OtBu werden in 100 ml DMF gelöst und dann nacheinander 4.6 g (25mMol) Thyminylessigsäure, 8.2 g(25mMol) TOTU und 8.5 ml (100mMol) Diisopropylethylamin zugegeben. Die Mischung wird 4h bei Raumtemperatur gerührt und dann im Vakuum eingedampft. Der ölige Rückstand wird in 350 ml Ethylacetat aufgenommen und jeweils 5mal mit je 5 ml Natriumhydrogencarbonat-Lösung bzw. Kaliumhydrogensulfat-Lösung extrahiert. Dann wird noch dreimal mit je 5 ml Wasser gewaschen. Hierbei fällt schon Substanz in der organischen Phase aus, die abgesaugt wird. Die organische Phase des Filtrats wird abgetrennt, über Natriumsulfat getrocknet und dann zur Trockene eingeengt. Der verbliebene feste Rückstand wird mit Diethylether verrieben, abgesaugt und getrocknet. Die vereinigten Rohprodukte werden aus Ethylacetat umkristallisiert.
Ausbeute: 9.71 g
MS(ES⁺): 441.3 (M+H)⁺
R_{f} =0.55 (Dichlormethan:Methanol:Triethylamin/100:10:1)

### Beispiel 13:

### N-(tert.Butyloxycarbonylaminoethyl)-N-((1-(N⁴-(4-Methoxylbenzoyl)-cytosyl)acetyl)glycin-tert.butylester

### Boc-Aeg(C^{MeBz})-OtBu

1.375 g (5 mMol) Boc-Aeg-OtBu werden in 20 ml DMF gelöst und dann nacheinander 1.515 g (5mMol) N⁴-(4-methoxybenzoyl)-N¹-carboxymethylcytosin, 1.64 g(5mMol) TOTU und 1.7 ml (10mMol) Diisopropylethylamin zugegeben. Die Mischung wird über Nacht bei Raumtemperatur gerührt und dann im Vakuum eingedampft. Der halbfeste Rückstand wird in Dichlormethan aufgenommen, filtriert und das Filtrat eingeengt. Der Rückstand wird mit Ethylacetat verrieben, wobei das Produkt als Festsubstanz ausfällt. Es wird aus wenig Ethylacetat umkristallisiert, abgesaugt, mit Ethylacetat gewaschen und im Vakuum getrocknet.
Ausbeute: 1.89 g
MS(ES⁺): 560.3 (M+H)⁺
R_{f} = 0.53 (Dichlormethan:Methanol:Triethylamin/100:10:1)

### Beispiel 14:

### N-(tert.Butyloxycarbonylaminoethyl)-N-((9-(N⁶-4-methoxybenzoyl)-adenosyl)acetyl)glycin-tert.butylester

### Boc-Aeg(A^{MeBz})-OtBu

549 mg (2 mMol) Boc-Aeg-OtBu werden in 10 ml DMF gelöst und dann nacheinander 655 mg (2mMol) N⁶-(4-Methoxybenzoyl)-N⁹-carboxymethyladenin, 656 mg (2mMol) TOTU und 0.68 ml (4mMol) Diisopropylethylamin zugegeben. Die Mischung wird über Nacht bei Raumtemperatur gerührt und dann im Vakuum eingedampft. Der Rückstand wird in 30 ml Ethylacetat aufgenommen und jeweils 2mal mit je 5 ml Natriumhydrogencarbonat-Lösung bzw. Wasser gewaschen. Die organische Phase wird über Natriumsulfat getrocknet und dann zur Trockene eingeengt. Der verbliebene feste Rückstand wird mit Diethylether verrieben, abgesaugt und getrocknet. Ausbeute: 730 mg
MS(FAB, MeOH/NBA): 584.2 (M+H)⁺
R_{f} = 0.74 (Dichlormethan:Methanol /10:1 und 1% Triethylamin)

### Beispiel 15:

### N-(Amino)ethyl-N-((1-thyminyl)acetyl)glycin

### (H-Aeg(T)-OH)

11.0 g (25 mMol) Boc-Aeg(T)-OtBu werden mit 110 ml 95% Trifluoressigsäure (5% Wasser) versetzt. Es ensteht ein klare Lösung, die 2h bei Raumtemperatur gerührt wird. Das Reaktionsgemisch wird dann eingeengt und mit Diethylether verrieben. Das ausgefallene Rohprodukt wird direkt in den nächsten Syntheseschritt eingesetzt.
Ausbeute: 7.1 g amorpher Feststoff
MS(ES⁺): 285.2 (M+H)⁺
R_{f} =0.14 (2-Butanon:Wasser:Pyridin:Essigsäure/70:15:15:2)

### Beispiel 16:

### N-((4-Methoxyphenyl)-diphenylmethylamino)ethyl-N-((1-thyminyl)acetyl) glycin

### (Mmt-Aeg(T))-OH

7.1 g H-Aeg(T)-OH aus der voherigen Reaktion werden in 300 ml DMF gelöst, dann werden 13.9 ml (100 mMol) Triethylamin zugegeben gefolgt von 8.9 g (29.2 mMol) Mmt-CI in 5 Portionen. Die Mischung wird über Nacht gerührt, dann von Ungelöstem (unumgesetztes H-Aeg(T)-OH, 2.78 g) abfiltriert und das Filtrat im Vakuum eingedampft. Der Rückstand wird in 300 ml Dichlormethan aufgenommen dreimal mit 30 ml Wasser gewaschen. Die organische Phase wird über Natriumsulfat getrocknet und dann zur Trockene eingeengt. Der verbliebene feste Rückstand wird in wenig Ethylacetat gelöst, mit ein wenig Triethylamin versetzt und in 200 ml Diethylether eingerührt. Das ausgefallene Produkt wird abgesaugt, mit wenig Ether gewaschen und im Vakuum getrocknet.
Ausbeute: 7.3 g
MS(FAB, MeOH/NBA/LiCl): 569.5 (M+Li)⁺
R_{f} = 0.25 (Dichlormethan:Methanol:Triethylamin/100:10:1)

### Beispiel 17:

### N-(Amino)ethyl-N-((1-(N⁴-(4-Methoxylbenzoyl)-cytosyl)acetyl)glycin

### (H-Aeg(C^{MeBz})-OH)

1.6 g (2.9 mMol) Boc-Aeg(C^{MeBz})-OtBu werden mit einer Mischung aus 30 ml Dichlormethan und 16 ml 95% Trifluoressigsäure (5% Wasser) versetzt. Es entsteht eine klare Lösung, die 3h bei Raumtemperatur gerührt wird. Das Reaktionsgemisch wird dann eingeengt und mit Diethylether verrieben. Das ausgefallene Rohprodukt wird direkt in den nächsten Syntheseschritt eingesetzt.
Ausbeute: 1.86 g amorphe Festsubstanz
R_{f} = 0.34 (2-Butanon:Wasser:Pyridin:Essigsäure/70:15:15:2)

### Beispiel 18:

### N-((4-Methoxyphenyl)-diphenylmethylamino)ethyl-N-((1-(N⁴-(4-Methoxylbenzoyl)-cytosyl)acetyl) glycin

### (Mmt-Aeg(C^{MeBz}))-OH

1.26 g H-Aeg(C^{MeBz})-OH aus der voherigen Reaktion werden in 50 ml DMF gelöst, dann werden 2.1 ml (15 mMol) Triethylamin zugegeben gefolgt von 1.06 g (3.5 mMol) Mmt-CI in 3 Portionen. Die Mischung wird über Nacht gerührt, dann von wenig Ungelöstem abfiltriert und das Filtrat im Vakuum eingedampft. Der Rückstand wird in 50 ml Dichlormethan aufgenommen dreimal mit 10 ml Wasser gewaschen. Die organische Phase wird über Natriumsulfat getrocknet, dann auf ca. 10 ml eingeengt und in 80 ml Diethylether eingerührt. Das ausgefallene Produkt wird abgesaugt, mit wenig Ether gewaschen und im Vakuum getrocknet.
Ausbeute: 1.35 g
MS(FAB, MeOH/NBA): 676.4 (M + H)⁺
R_{f} = 0.62 (2-Butanon:Wasser:Pyridin:Essigsäure/70:15:15:2)

### Beispiel 19:

### N-(Amino)ethyl-N-((9-(N⁶-4-methoxybenzoyl)-adenosyl)acetyl)glycin

### (H-Aeg(A^{MeBz})-OH)

725 mg (1.2mMol) Boc-Aeg(A^{MeBz})-OtBu werden mit 10 ml 95% Trifluoressigsäure (5% Wasser) versetzt. Es entsteht ein klare Lösung, die 3h bei Raumtemperatur gerührt wird. Das Reaktionsgemisch wird dann eingeengt und mit Diethylether verrieben. Das ausgefallene Rohprodukt wird direkt in den nächsten Syntheseschritt eingesetzt.
Ausbeute: 830 mg amorphe Festsubstanz

### Beispiel 20:

### N-((4-Methoxyphenyl)-diphenylmethylamino)ethyl-N-((9-(N⁶-4-methoxybenzoyl)-adenosyl)acetyl) glycin

### (Mmt-Aeg(A^{MeBz}))-OH

690 mg H-Aeg(A^{MeBz})-OH aus der voherigen Reaktion werden in 25 ml DMF gelöst, dann werden 0.88 ml (6.5 mMol) Triethylamin zugegeben gefolgt von 494 mg (1.6 mMol) Mmt-CI in 3 Portionen. Die Mischung wird über Nacht gerührt, dann von wenig Ungelöstem abfiltriert und das Filtrat im Vakuum eingedampft. Der Rückstand wird in 20 ml Dichlormethan aufgenommen dreimal mit 5 ml Wasser gewaschen. Die organische Phase wird über Natriumsulfat getrocknet, dann eingeengt und der Rückstand mit Diethylether verrieben. Das ausgefallene Produkt wird abgesaugt, mit wenig Ether gewaschen und im Vakuum getrocknet.
Ausbeute: 440 mg
MS(ES⁺): 700.3 (M+H)⁺
R_{f}=0.59 (2-Butanon:Wasser:Pyridin:Essigsäure/70:15:15:2)

### Beispiel 21:

### N-(tert.Butyloxycarbonylaminoethyl)- N-((9-(N²-acetyl-O⁴-diphenylcarbamoyl)-guanosyl)acetyl)-glycin-tert.butylester Boc-Aeg(G^{2-Ac,4-Dpc})-OtBu

1.375 g (5mMol) Boc-Aeg-OtBu werden in 30 ml DMF gelöst und dann nacheinander 2.23 g (5mMol) N²-Acetyl O⁴-diphenylcarbamoyl-9-carboxymethyl-guanin, 1.64 g(5mMol) TOTU und 1.7 ml (10mMol) Diisopropylethylamin zugegeben. Die Mischung wird über Nacht bei Raumtemperatur gerührt und dann im Vakuum eingedampft. Der Rückstand wird in 50 ml Ethylacetat aufgenommen, je 4mal mit 3 ml Natriumhydrogencarbonat-Lösung, 3 ml Kaliumhydrogensulfat-Lösung und 3 ml Wasser extrahiert. Die organische Phase wird über Natriumsulfat getrocknet, das Trockenmittel abfiltriert und das Filtrat eingeengt. Der Rückstand wird mit Ether verrieben, wobei das Produkt als harzige Festsubstanz zurückbleibt. Dieses Produkt wird ohne weitere Reinigung in die nachfolgende Reaktion eingesetzt
Ausbeute:3.14 g
MS(FAB, NBA/MeOH): 703.3 (M+H)⁺
R_{f} = 0.80(Dichlormethan:Methanol:Triethylamin/100:10:1

### Beispiel 22:

### N-(aminoethyl)-N-((9-(N²-acetyl)guanosyl)acetyl)-glycin

### H-Aeg(G^{2-Ac})-OH

2.08 g (2.96 mMol) Boc-Aeg(G^{2-Ac,4-Dpc})-OtBu werden mit 20 ml 95% Trifluoressigsäure (5% Wasser) versetzt. Es ensteht ein klare Lösung, die 30min bei Raumtemperatur gerührt wird. Das Reaktionsgemisch wird dann eingeengt und mit Diethylether verrieben. Das ausgefallene Rohprodukt wird direkt in den nächsten Syntheseschritt eingesetzt.
Ausbeute: 1.46 g amorphe Festsubstanz
R_{f} = 0.15(2-Butanon:Wasser:Pyridin:Essigsäure/70:15:15:2)

### Beispiel 23:

### Mmt-Aeg(G^{2-Ac})-OH

1.46g H-Ae(G^{2-Ac})-OH aus der voherigen Reaktion werden in 30 ml DMF gelöst, dann werden 2.1 ml (15mMol) Triethylamin zugegeben gefolgt von 939 mg (3.04mMol) Mmt-Cl in 3 Portionen. Die Mischung wird über Nacht gerührt, dann von wenig Ungelöstem abfiltriert und das Filtrat im Vakuum eingedampft. Der Rückstand wird in 30 ml Ethylacetat aufgenommen, je 3mal mit 3 ml Natriumhydrogencarbonat-Lösung und 3 ml Wasser extrahiert. Die organische Phase wird über Natriumsulfat getrocknet, das Trockenmittel abfiltriert und das Filtrat eingeengt. Der Rückstand wird mit Ether verrieben. Das ausgefallene Produkt wird abgesaugt, mit wenig Ether gewaschen und im Vakuum getrocknet.
Ausbeute: 1.10 g
MS(ES⁺): 624.3 (M+H)⁺
R_{f} = 0.14(Dichlormethan:Methanol:Triethylamin/100:10:1)

### Beispiel 24:

### N-(tert.Butyloxycarbonylaminoethyl)-N-((1-(N⁴-(4-Methoxylbenzoyl)-cytosyl)acetyl)glycin-tert.butylester

### Boc-Aeg(C^{MeOBz})-OtBu

114.6 g (0.418 Mol) Boc-Aeg-OtBu und 127 g(0.418 Mol) N⁴-(4-methoxybenzoyl)-N-carboxymethylcytosin werden mit 1.5 l Ethylacetat versetzt und zur gerührten Suspension 115 ml (0.836 Mol) Triethylamin hinzugegeben. Dann werden werden 400 ml (ca. 0.628 Mol) einer 50%igen Propanphosphonsäureanhydrid-Lösung in Ethylacetat innerhalb von 2 min zugegeben. Hierbei steigt die Temperatur leicht an. Die Mischung wird durch Zugabe von weiteren 65 ml (0.469 Mol) Triethylamin auf pH 8 gestellt und noch 2.5 h nachgerührt. Der Niederschlag wird abgesaugt, zuerst mit 500 ml Ethylacetat und dann mit 1 I Wasser gewaschen. Aus der Mutterlauge fällt noch weiteres Produkt aus. Beide Niederschläge werden erneut mit 500 ml Wasser verrührt, abgesaugt, mit 500 ml Wasser gewaschenund im Vakuum getrocknet. Ausbeute: 196.6 g

### Beispiel 25:

### N-(tert.Butyloxycarbonylaminoethyl)-N-((9-(N⁶-4-methoxybenzoyl)-adenosyl)acetyl)glycin-tert.butylester

### Boc-Aeg(A^{MeOBz})-OtBu

300.0 g (1.094 Mol) Boc-Aeg-OtBu werden in 2.8 I DMF gelöst und dann nacheinander 358.2 g ( 1.094 Mol) N⁶-(4-Methoxybenzoyl)-N⁹-carboxymethyladenin, 360.5 g (2mMol) TOTU und 373.9 ml (2.184 Mol) Diisopropylethylamin zugegeben. Die Mischung wird noch 2h bei Raumtemperatur gerührt und dann in eine gerührte eisgekühlte Lösung von 214.29 g Natriumhydrogencarbonat in 2.2 I Wasser getropft, wobei das Produkt ausfällt. Anschließend werden noch 2 I Methyl-tert.butylether zugegeben. Diese Mischung wird über Nacht stehengelassen. Dann wird der Nieder schlag abgesaugt und nacheinander mit je zweimal 2I Wasser und 2I Methyl-tert.butylether verrührt, erneut abfiltriert und der Niederschlag im Vakuum getrocknet. Ausbeute: 437.7 g

### Beispiel 26:

### N-(tert.Butyloxycarbonylaminoethyl)glycin-tert.butylester

### Boc-Aeg-OtBu

### Stufe 1:N-Boc-1,2-Diaminoethan

535 ml (8 Mol) 1,2-Diaminoethan werden in 2 I Dichlormethan gelöst. Unter Rühren und Kühlung wird eine Lösung von 218.5 g (1 Mol) Di-tert.-butyldicarbonat in 500 ml Dichlormethan innerhalb von 1.5 h zugetropft. Die Mischung wird noch 20 h nachgerührt, dann mit 1l Wasser versetzt und kräftig durchgerührt. Die Phasen werden getrennt und die Wasserphase noch zweimal mit je 500 ml Dichlormethan extrahiert. Die vereinigten organischen Phasen werden mit halbgesättigter Natriumchloridlösung gewaschen und über Natriumsulfat getrocknet. Die organische Phase wird auf 1 l Volumen eingeengt

### Stufe 2: Boc-Aeg-OtBu

Zu der oben erhaltenen Lösung gibt man 138 ml (0.97 Mol) Triethylamin und eine Spatelspitze Kaliumjodid. Dann wird unter Rühren eine Lösung von 149.5 ml Chloressigsäure-tert.-butylester zugetropft und diese Mischung 50 h bei 40°C gerührt. Dann werden 500 ml Wasser zugegeben, die Mischung kräftig durchgerührt und die organische Phase abgetrennt, über Natriumsulfat getrocknet und eingeengt. Das erhaltene Rohprodukt wird an Kieselgel mit Ethylacetat Methanol 95:5 als Eluent chromatographisch gereinigt. Man erhält 78 g des gewünschten Produktes.

### Beispiel 27:

### N-(tert.Butyloxycarbonylaminoethyl)glycin-tert.butylester

### Boc-Aeg-OtBu

### Stufe 1:N-Boc-1,2-Diaminoethan

4.8 I (72 Mol) 1,2-Diaminoethan werden in 18 I Dichlormethan gelöst. Unter Rühren und Kühlung wird eine Lösung von 1.97 kg (9.03 Mol) Di-tert.-butyldicarbonat in 4.5 I Dichlormethan innerhalb von 2 h zugetropft. Die Mischung wird noch 20 h nachgerührt, dann mit 9l Wasser versetzt und kräftig durchgerührt. Die Phasen werden getrennt und die Wasserphase noch zweimal mit je 4.5 l Dichlormethan extrahiert. Die vereinigten organischen Phasen werden mit halbgesättigter Natriumchloridlösung gewaschen und über Natriumsulfat getrocknet. Die organische Phase wird auf 9 I Volumen eingeengt

### Stufe 2: Boc-Aeg-OtBu

Zu der oben erhaltenen Lösung gibt man 1.24 I Triethylamin und 5g Kaliumjodid. Dann wird unter Rühren eine Lösung von 1.245 l Chloressigsäure-tert.-butylester in 4.5 I Dichlormethan zugetropft und diese Mischung 50 h bei 40°C gerührt. Dann werden 5 I Wasser zugegeben, die Mischung kräftig durchgerührt und die organische Phase abgetrennt, über Natriumsulfat getrocknet und eingeengt. Das erhaltene Rohprodukt wird an Kieselgel mit Ethylacetat Methanol 95:5 als Eluent chromatographisch gereinigt. Man erhält 1.03 kg des gewünschten Produktes.

Weitere verwendete Abkürzungen sind nachfolgend aufgelistet.
- Aeg: N-(2-Aminoethyl)glycyl, -NH-CH₂-CH₂-NH-CH₂-CO-
- Aeg(A^{MeOBz}): N-(2-Aminoethyl)-N-((9-(N⁶-4-methoxybenzoyl)-adenosyl)acetyl)-glycyl
- Aeg(C^{Bz}): N-(2-Aminoethyl)-N-((1-(N⁴-benzoyl)-cytosyl)acetyl)-glycyl
- Aeg(C^{MeOBz}): N-(2-Aminoethyl)-N-((1-(N⁴-4-methoxybenzoyl)-cytosyl)acetyl)-glycyl
- Aeg(C^{tBuBz}): N-(2-Aminoethyl)-N-((1-(N⁴-4-tert.butylbenzoyl)-cytosyl)acetyl)-glycyl
- Aeg(G^{iBu}): N-(2-Aminoethyl)-N-((9-(N²-isobutanoyl)-guanosyl)acetyl)-glycyl
- Aeg(G^{2-Ac,4-Dpc}): N-(2-Aminoethyl)-N-((9-(N²-acetyl-O⁴⁻ diphenylcarbamoyl)guanosyl)acetyl)-glycyl
- Aeg(G^{2-Ac}): N-(2-Aminoethyl)-N-((9-(N²-acetyl)-guanosyl)acetyl)-glycyl
- Aeg(T): N-(2-Aminoethyl)-N-((1-thyminyl)acetyl)-glycyl
- Bnpeoc: 2,2-[Bis(4-nitrophenyl)]-ethoxycarbonyl)
- Boc: tert.-Butyloxycarbonyl
- BOI: 2-(Benzotriazol-1-yl)oxy-1,3-dimethyl-imidazolidinium-hexafluorphosphat
- BOP: Benzotriazolyl-1-oxy-tris(dimethylamino)-phosphonium-hexafluorphosphat
- BroP: Brom-tris(dimethylamino)-phosphonium-hexafluorphosphat
- BSA: N,O-Bis-(trimethylsilyl)-acetamid
- But: tert.-Butyl
- Bz: Benzoyl
- Bzl: Benzyl
- Cl-Z: 4-Chlor-benzyloxycarbonyl
- CPG: Controlled Pore Glas
- DBU: 1,8-Diazabicyclo[5.4.0]undec-7-en
- DCM: Dichlormethan
- Ddz: 3.5-Dimethoxyphenyl-2-propyl-2-oxycarbonyl
- DMF: Dimethylformamid
- Dmt: Di-(4-methoxyphenyl)phenylmethyl,
- Dnpeoc: 2-(2,4-Dinitrophenyl)-ethoxycarbonyl
- Dpc: Diphenylcarbamoyl
- FAM: Fluorescein-Rest
- Fm: 9-Fluorenylmethyl
- Fmoc: 9-Fluorenylmethyloxycarbonyl
- H-Aeg-OH: N-(2-Aminoethyl)glycin
- HAPyU: O-(7-Azabenzotriazol-1 -yl)-1,1,3,3-bis(tetramethylen)uronium-hexafluorphosphat
- HATU: O-(7-Azabenzotriazol-1-yl)-1,1,3,3-tetramethyluronium-hexafluorphosphat
- HBTU: O-(Benzotriazol-1-yl)-1,1,3,3-tetramethyluronium-hexafluorphosphat
- HOBt: 1-Hydroxybenzotriazol
- HONSu: N-Hydroxysuccinimid
- HOObt: 3-Hydroxy-4-oxo-3,4-dihydrobenzotriazin
- iBu: Isobutanoyl
- MeOBz: 4-Methoxybenzoyl
- Mmt: 4-Methoxytriphenylmethyl
- Moz: 4-Methoxybenzyloxycarbonyl
- MSNT: 2,4,6-Mesitylensulfonyl-3-nitro-1,2,4-triazolid
- Mtt: 4-Methylphenyl)diphenylmethyl
- NEt₃: Triethylamin
- NMP: N-Methylpyrrolidin
- Oeg: N-(2-Oxyethyl)glycyl, -O-CH₂-CH₂-NH-CH₂-CO-
- Pixyl: 9-(9-Phenyl)xanthenyl
- PyBOP: Benzotriazolyl-1-oxy-tripyrrolidinophosphonium-hexafluorphosphat
- PyBroP: Brom-tripyrrolidinophosphonium-hexafluorphosphat
- TAPipU: O-(7-Azabenzotriazol-1-yl)-1,1,3,3-bis(pentamethylen)uronium-tetrafluoroborat
- TBTU: O-(Benzotriazol-1-yl)-1,1,3,3-tetramethyluronium-tetrafluoroborat
- tBu: tert.-Butyl
- tBuBz: 4-tert. Butylbenzoyl
- TDBTU: O-(3,4-Dihydro-4-oxo-1,2,3-benzotriazin-3-yl)-1,1,3,3-tetramethyluronium-tetrafluoroborat
- TDO: 2,5-Diphenyl-2,3-dihydro-3-oxo-4-hydroxythiophendioxid
- TFA: Trifluoressigsäure
- THF: Tetrahydrofuran
- TNTU: O-[(5-Norbonen-2,3-dicarboximido]-1,1,3,3-tetramethyluronium-tetrafluoroborat
- TOTU: O-[(Cyano(ethoxycarbonyl)methylen)amino]-1,1,3,3-tetramethyluronium-tetrafluoroborat
- TPTU: O-(1,2-Dihydro-2-oxo-1-pyridyl)-1,1,3,3'-tetramethyluroniu m-tetrafluoroborat
- Trt: Trityl
- TSTU: O-(N-Succinimidyl)-1,1,3,3-tetramethyluronium-tetrafluoroborat
- Z: Benzyloxycarbonyl
- MS(ES⁺): Elektrospray Massenspektrum (Positiv Ion)
- MS(ES⁻): Elektrospray Massenspektrum (Negativ Ion)
- MS(DCI): Direkt-Ionisations-Massenspektrum
- MS(FAB): East-Atom-Bombardement-Massenspektrum
- NBA: Nitrobenzylalkohol

## Patentansprüche

1. Verfahren zur Herstellung substituierter N-Ethyl-Glycinderivate der Formel I 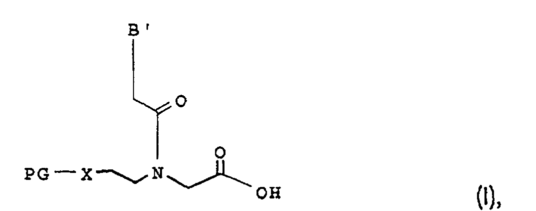 worin
PG für eine gegen schwache Säuren labile Aminoschutzgruppe vom Urethantyp oder vom Trityl-Typ,
X für NH oder O, und
B' für natürliche oder unnatürliche Basen, deren exocyclische Amino bzw. Hydroxygruppen durch Schutzgruppen geschützt sind, stehen,
sowie deren Salze,
**dadurch gekennzeichnet, daß** man
a) eine Verbindung der Formel II 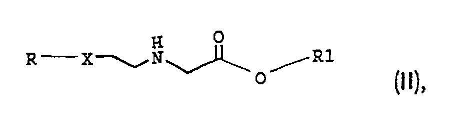 worin
R Wasserstoff, oder wenn X = NH ist, eine säurelabile Schutzgruppe und
R¹ eine gegen Säuren labile und gegen Amine stabile Schutzgruppe
bedeuten, und
X wie oben definiert ist,
mit einer Verbindung der Formel III 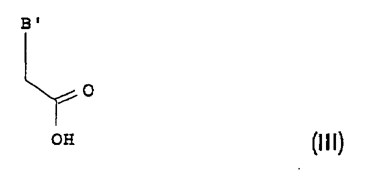 worin
B' wie oben definiert ist,
bei 0 - 45°C in einem Lösungsmittel unter Verwendung eines Kupplungsreagenzes
zu einer Verbindung der Formel IV 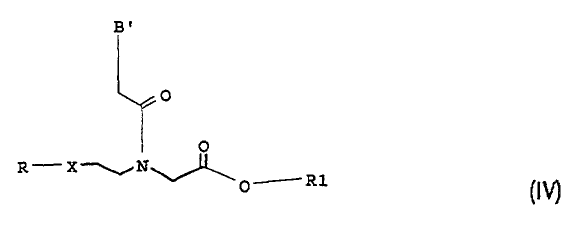 worin
R, X, B' und R¹ wie oben definiert sind,
umsetzt,
b) anschließend die Verbindung der Formel IV durch Abspaltung der säurelabilen Esterschutzgruppe R¹ bzw. für X = NH durch gleichzeitige Abspaltung der säurelabilen Schutzgruppe R unter sauren Bedingungen in einem Lösungsmittel gegebenenfalls unter Zusatz von Kationenfängern in eine Verbindung der Formel V überführt, wobei Verbindungen der Formel IV mit X = O gegebenenfalls zusätzlich mit Basen in wäßrigem Medium behandelt werden, 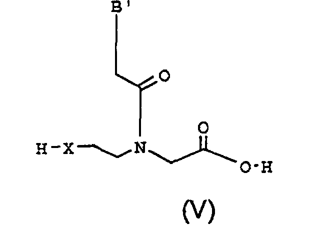 worin
X und B' wie oben definiert sind,
c) die Verbindung der Formel V mit einem Reagenz in einem Lösungsmittel unter Verwendung einer Hilfsbase in die Verbindung der Formel I und anschließend gegebenenfalls in deren Salze überführt.

2. Verfahren zur Herstellung substituierter N-Ethyl-Glycinderivate der Formel I gemäß Anspruch 1, **dadurch gekennzeichnet, daß** man
a) eine Verbindung der Formel II,
worin
R Wasserstoff, oder wenn X = NH ist, Boc, Ddz oder Trt und
R¹ tert. Butyl oder (2-Chlorphenyl)diphenylmethyl bedeuten, und
X wie oben definiert ist,
mit einer Verbindung der Formel III,
worin
B' wie oben definiert ist,
bei Raumtemperatur, in DMF, Acetonitril, Dichlormethan oder Mischungen dieser Lösungsmittel unter Verwendung von Carbodiimiden, Phosphonium-Reagenzien, Uronium-Reagenzien, Säurehalogeniden oder aktivierten Estem,
zu einer Verbindung der Formel IV
worin
R, X, B' und R¹ wie oben definiert sind,
umsetzt,
b) anschließend die Verbindung der Formel IV durch Abspaltung der säurelabilen Esterschutzgruppe R¹ bzw. für X = NH durch gleichzeitige Abspaltung der säurelabilen Schutzgruppe R mit Trifluoressigsäure in Dichlormethan, Ethylacetat oder Dioxan gegebenenfalls unter Zusatz von Anisol, Thiophenol oder Triethylsilan
in eine Verbindung der Formel V überführt,
worin
X und B' wie oben definiert sind, und
wobei die Verbindungen der Formel IV mit X = O gegebenenfalls zusätzlich mit NaOH oder Triethylamin in wäßrigem Medium behandelt werden,
c) die Verbindung der Formel V mit t-Bumeoc-fluorid, Adpoc-azid, Bpoc-azid, Ddz-(phenyl)carbonat, Trt-Cl, Mtt-Cl, Mmt-Cl, Dmt-CI oder Pixyl-Cl in DMF, NMP, Acetonitril, Dichlormethan oder Mischungen dieser Lösungsmittel unter Verwendung von NEM, DIPEA, Pyridin, Triethylamin in die Verbindung der Formel I und anschließend gegebenenfalls in deren Salze überführt.

3. Verfahren zur Herstellung substituierter N-Ethyl-Glycinderivate der Formel I gemäß Anspruch 2, **dadurch gekennzeichnet, daß** in der Verbindung der Formel II R Wasserstoff oder wenn X = NH ist, Boc und R¹ tert. Butyl sind.

4. Zwischenprodukt der Formel IVa, 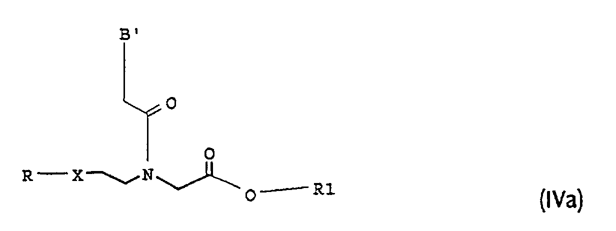 worin
X für NH oder O,
B' für in der Nucleotidchemie übliche Basen, deren exocyclische Amino bzw. Hydroxygruppen durch geeignete bekannte Schutzgruppen geschützt sind, stehen,
R Wasserstoff, oder wenn X = NH ist, eine säurelabile Schutzgruppe und
R¹ tert. Butyl oder (2-Chlorphenyl)diphenylmethyl
bedeuten.

## Claims

1. A process for preparing substituted N-ethylglycine derivatives of the formula I 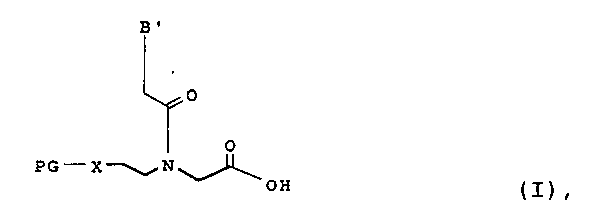 in which
PG is an amino protecting group which is labile towards weak acids and which is of the urethane type or the trityl type,
X is NH or O, and
B' is natural or unnatural bases whose exocyclic amino and/or hydroxyl groups are protected by protecting groups,
and the salts thereof,
which comprises
a) reacting a compound of the formula II 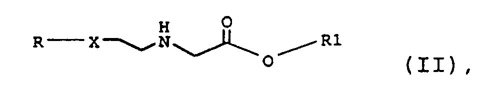 in which
R is hydrogen or, if X = NH, an acid-labile protecting group, and
R¹ is a protecting group which is labile towards acids and stable towards amines, and
X is defined as above,
with a compound of the formula III 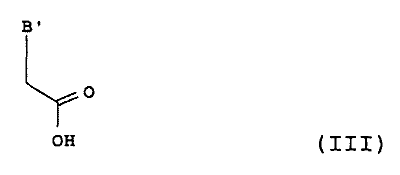 in which
B' is defined as above,
at 0 - 45°C in a solvent using a coupling reagent
to give a compound of the formula IV 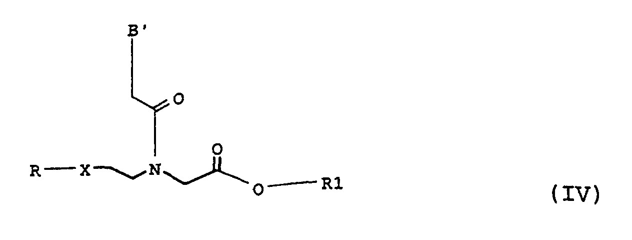 in which
R, X, B' and R¹ are defined as above,
b) subsequently converting the compound of the formula IV, by means of eliminating the acid-labile ester protecting group R¹ and, for X = NH, by means of simultaneously eliminating the acid-labile protecting group R under acidic conditions, in a solvent, where appropriate with the addition of cation-capturing agents, into a compound of the formula V, with compounds of the formula IV in which X = O being, where appropriate, additionally treated with bases in aqueous medium, 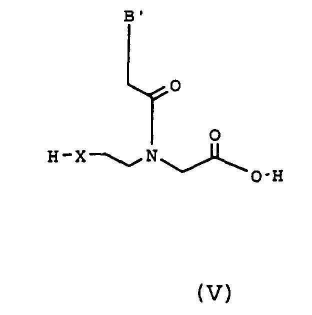 in which
X and B' are defined as above,
c) converting the compound of the formula V, using a reagent in a solvent and using an auxiliary base, into the compound of the formula I and subsequently, where appropriate, converting the latter into its salts.

2. The process for preparing substituted N-ethylglycine derivatives of the formula I as claimed in claim 1, wherein
a) a compound of the formula II,
in which
R is hydrogen, or, if X = NH, Boc, Ddz or Trt and
R¹ is tert-butyl or (2-chlorophenyl)diphenylmethyl, und
X is defined as above,
is reacted with a compound of the formula III,
in which
B' is defined as above,
at room temperature, in DMF, acetonitrile, dichloromethane or mixtures of these solvents, using carbodiimides, phosphonium reagents, uronium reagents, acid halides or activated esters,
to give a compound of the formula IV
in which
R, X, B' and R¹ are defined as above,
b) the compound of the formula IV is subsequently converted, by means of eliminating the acid-labile ester protecting group R¹ and, for X = NH, by means of simultaneously eliminating the acid-labile protecting group R with trifluoroacetic acid in dichloromethane, ethyl acetate or dioxane, where appropriate with the addition of anisole, thiophenol or triethylsilane,
into a compound of the formula V,
in which
X and B' are defined as above, and
with the compounds of the formula IV in which X = O being additionally treated, where appropriate, with NaOH or triethylamine in aqueous medium,
c) the compound of the formula V is converted, with t-Bumeoc fluoride, Adpoc azide, Bpoc azide, Ddz-(phenyl)carbonate, Trt-Cl, Mtt-Cl, Mmt-Cl, Dmt-Cl or Pixyl-Cl in DMF, NMP, acetonitrile, dichloromethane or mixtures of these solvents, using NEM, DIPEA, pyridine or triethylamine, into the compound of the formula I and subsequently, where appropriate, into its salts.

3. The process for preparing substituted N-ethylglycine derivatives of the formula I as claimed in claim 2, wherein, in the compound of the formula II, R is hydrogen or, if X = NH, Boc, and R¹ is tert-butyl.

4. An intermediate of the formula IVa, 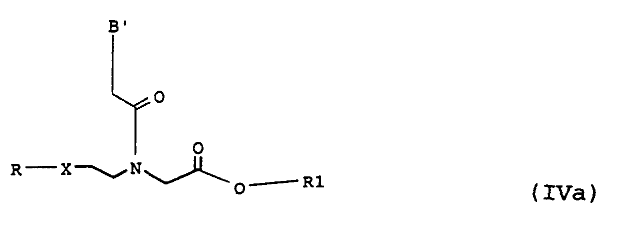 in which
X is NH or O,
B' is bases which are customary in nucleotide chemistry and whose exocyclic amino and/or hydroxyl groups are protected by suitable known protecting groups,
R is hydrogen or, if X = NH, an acid-labile protecting group, and
R¹ is tert-butyl or (2-chlorophenyl) diphenylmethyl.

## Revendications

1. Procédé pour la préparation de dérivés substitués de N-éthyl-glycine de formule I 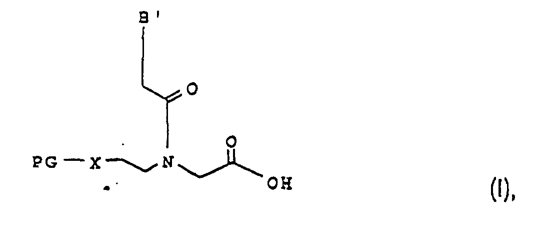 dans laquelle
PG représente un groupe de protection amino labile vis à vis des acides faibles de type uréthane, ou de type trityle,
X représente NH ou O, et
B' représente des bases naturelles ou artificielles, dont les groupes amino ou hydroxy exocycliques sont protégés par des groupes de protection,
ainsi que leurs sels,
**caractérisé en ce qu'**on met à réagir
a) un composé de formule II 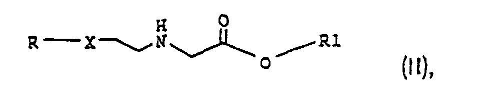 dans laquelle
R représente un atome d'hydrogène, ou lorsque X = NH, un groupe de protection labile en milieu acide, et
R¹ représente un groupe de protection labile vis à vis des acides et stable vis à vis des amines, et
X est défini comme ci-dessus,
avec un composé de formule III 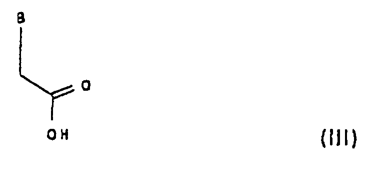 dans laquelle
B' est défini comme ci-dessus,
à 0-45°C dans un solvant en utilisant un réactif de couplage
pour obtenir un composé de formule IV 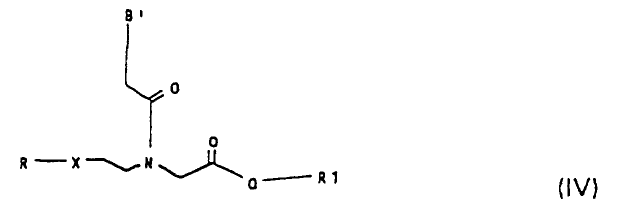 dans laquelle
R, X, B' et R¹ sont définis comme ci-dessus,
b) on transforme ensuite le composé de formule IV par coupure du groupe de protection ester labile en milieu acide R¹ ou pour X = NH par coupure simultanée du groupe de protection R labile en milieu acide dans des conditions acides et dans un solvant approprié en ajoutant éventuellement des fixateurs de cations, en un composé de formule V, opération dans laquelle des composés de formule IV avec X = O sont éventuellement traités additionnellement avec des bases en milieu aqueux, 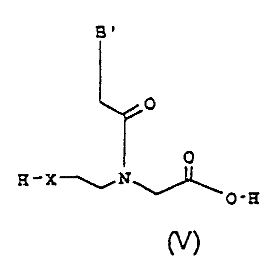 dans lesquelles
X et B' sont définis comme ci-dessus.
c) on transforme le composé de formule V avec un réactif dans un solvant en utilisant une base additionnelle pour obtenir le composé de formule I et ensuite éventuellement ses sels.

2. Procédé pour la préparation de dérivés de N-éthyl-glycine de formule I selon la revendication 1,
**caractérisé en ce qu'**on met à réagir
a) un composé de formule II,
dans laquelle
R représente un atome d'hydrogène, ou lorsque X = NH, Boc, Ddz ou Trt et
R¹ représente un groupe tert.-butyle ou (2-chlorophényl)diphénylméthyle, et
X est défini comme ci-dessus,
avec un composé de formule III,
dans laquelle
B' est défini comme ci-dessus,
à température ambiante, dans le DMF, l'acétonitrile, le dichlorométhane ou des mélanges de ces solvants en utilisant des carbodiimides, des réactifs phosphonium, des réactifs uronium, des halogénures d'acide ou des esters activés,
pour obtenir un composé de formule IV
dans laquelle
R, X, B' et R¹ sont définis comme ci-dessus.
b) on transforme ensuite le composé de formule IV par coupure du groupe de protection ester labile en milieu acide R¹ ou pour X = NH par coupure simultanée du groupe de protection labile en milieu acide R avec l'acide trifluoroacétique dans le dichlorométhane, l'acétate d'éthyle ou le dioxane éventuellement avec addition d'anisol, de thiophénol ou de triéthylsilane pour obtenir un composé de formule V,
dans laquelle
X et B' sont définis comme ci-dessus, et opération dans laquelle les composés de formule IV avec X = O sont éventuellement traités additionnellement avec NaOH ou la triéthylamine en milieu aqueux,
c) on transforme le composé de formule V avec t-Bumeoc-fluorure, Adpoc-azide, Bpoc-azide, Ddz-(phényl)carbonate, Trt-Cl, Mtt-Cl, Mmt-Cl, Dmt-Cl ou Pixyl-Cl dans le DMF, la NMP, l'acétonitrile, le dichlorométhane ou des mélanges de ces solvants en utilisant NEM, DIPEA, la pyridine, le triéthylamine pour obtenir le composé de formule I et on le transforme éventuellement ensuite en ses sels.

3. Procédé pour la préparation de dérivés substitués de N-éthyl-glycine de formule I selon la revendication 2, **caractérisé en ce que**, dans le composé de formule II R est un atome d'hydrogène ou lorsque X est NH, représente Boc et R¹ est le tert.-butyle.

4. Produit intermédiaire de formule IVa, 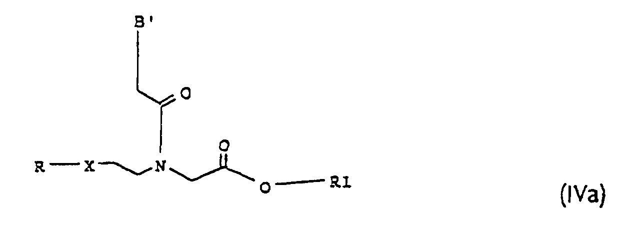 dans laquelle
X représente NH ou O,
B représente des bases usuelles dans la chimie des nucléotides, dont les groupes hydroxy ou amino exocycliques sont protégés par des groupes de protection appropriés connus,
R représente un atome d'hydrogène, ou lorsque X = NH, un groupe de protection labile en milieu acide et
R¹ représente un groupe tert.-butyle ou (2-chlorophényl)diphénylméthyle.
